# EUROPEAN PATENT APPLICATION

(11) **EP 4 821 921 A1**
(43) Date of publication of application: **14.10.2026**
(21) Application number: 23960694.0
(22) Date of filing: 05.12.2023
(51) Int. Cl.: C07D 241/18, C05G 3/90, C05C 13/00

(54) **NITRIFICATION INHIBITOR AND USE THEREOF IN FERTILISERS CONTAINING AMMONIACAL NITROGEN, UREA NITROGEN OR BOTH**

(71) Applicant: FERTINAGRO BIOTECH, S.L., 44195 Teruel (ES)
(72) Inventor: ATARES REAL, Sergio, 44195 TERUEL (ES); ROMERO LOPEZ, Joaquin, 44195 TERUEL (ES); SALAET MADORRAN, Ignasi, 44195 TERUEL (ES); CABALLERO MOLADA, Marcos, 44195 TERUEL (ES); VALLEJO GARCÍA, Antonio, 44195 TERUEL (ES); JIMENEZ HORCAJADA, Rafael, 44195 TERUEL (ES); DORADO HORRILLO, Vicente, 44195 TERUEL (ES); FRAILE DOLADO, José María, 44195 TERUEL (ES); YANCE CHÁVEZ, Tula Del Carmen, 44195 TERUEL (ES); JIMENO BRAVO, Inés, 44195 TERUEL (ES)
(74) Representative: González López-Menchero, Álvaro Luis
(86) International application number: PCT/ES2023/070732
(87) International publication number: WO 2025/120244

(57) **Abstract**

The present invention discloses a nitrification inhibitor for use thereof in fertilisers containing ammoniacal nitrogen, urea nitrogen or both, wherein the nitrification inhibitor is pulcherriminic acid. The pulcherriminic acid is present in a weight ratio of 0.5 to 10 % relative to the amount of ammoniacal nitrogen, urea nitrogen, or both ammoniacal nitrogen and urea nitrogen.

## Description

The present invention discloses a nitrification inhibitor for use thereof in fertilisers containing ammoniacal nitrogen, urea nitrogen or both, wherein the nitrification inhibitor is pulcherriminic acid, the pulcherriminic acid is present in a weight ratio of 0,5 to 10 % relative to the amount of ammoniacal nitrogen, urea nitrogen, or both ammoniacal nitrogen and urea nitrogen.

Currently, ammonium-based chemical fertilisers (NH₄⁺), such as urea, NH₃ anhydrous, (NH₄)₂SO₄ and NH₄NO₃, are the most widely used worldwide, as is the case with liquid manure, the major nitrogenous moiety of which is ammonium. This continuous supply of NH₄⁺ to the soil stimulates the activity of nitrifying microorganisms, so that most modern agricultural systems have become systems with high nitrification rates, to such an extent that nitrification is so rapid that most of the NH₄⁺ from external sources undergoes a complete nitrification process within a few weeks (Halvorson et al., Enhanced-Efficiency Nitrogen Fertilizers: Potential Role in Nitrous Oxide Emission Mitigation. Agronomy Journal 2014, 106, 715-722. https://doi.org/10.2134/agronj2013.0081; Subbarao et al., Scope and Strategies for Regulation of Nitrification in Agricultural Systems-Challenges and Opportunities. Critical Reviews in Plant Sciences 2006, 25, 303-335. https://doi.org/10.1080/07352680600794232).

The nitrification process is divided into two steps: the first step involves the oxidation of NH₃/NH₄⁺ to NO₂⁻, and a final one in which NO₂⁻ is oxidised to NO₃⁻ through the action of the enzyme nitrite oxidoreductase. In turn, the first step of nitrification is divided into two steps: the oxidation of NH₃/NH₄⁺ to hydroxylamine (NH₂OH) by the enzyme ammonium monooxygenase (AMO) and the oxidation of hydroxylamine to nitrite (NO₂⁻) by the enzyme hydroxylamine oxidoreductase (HAO). During this process nitrous oxide (N₂O) is formed due to the chemical decomposition of hydroxylamine.

The micro-organisms involved in nitrification are two groups of ammonium oxidisers, ammonium oxidising archaea (AOA) and ammonium oxidising bacteria (AOB). On the soil, the soil AOB communities are dominated by the genera *Nitrosomonas* and *Nitrosospira* within the *Betaproteobacteria.* The predominance of AOA or AOB in nitrification is related to soil pH and NH₄⁺ concentration. In acid soils, AOAs are the predominant group carrying out the nitrification process regardless of NH₄⁺ concentration, while AOBs become relevant as the pH reaches neutral or alkaline values. When NH₄⁺-based fertilisers are applied, AOA are inhibited and AOB carry out the nitrification process. As a result, alkaline and neutral soils tend to show an increase in AOB after fertilisation (Castellano-Hinojosa et al., Effect of Urease and Nitrification Inhibitors on Ammonia Volatilization and Abundance of N-cycling Genes in an Agricultural Soil. J. Plant Nutr. SoilSci. 2020, 183, 99-109. https://doi.org/10.1002/jpln.201900038).

The enzyme ammonium monooxygenase (AMO) consists of three different subunits (AmoA, AmoB and AmoC) and contains Cu (6 Cu²⁺ and 3 Cu⁺), Fe (4 Fe³⁺) and Zn (3 Zn²⁺) (Gilch et al., Electron Paramagnetic Studies of the Copper and Iron Containing Soluble Ammonia Monooxygenase from Nitrosomonas Europaea. Biometals 2010, 23, 613-622. https://doi.org/10.1007/s10534-010-9308-2). However, the AMO enzyme in AOA communities saturates faster than that in AOB communities due to a higher affinity for NH₄⁺ and a lower maximum NH₄⁺ oxidation rate. Therefore, although AOA are more abundant in agricultural soils (Di et al., Inhibition of Nitrification to Mitigate Nitrate Leaching and Nitrous Oxide Emissions in Grazed Grassland: A Review. J Soils Sediments 2016, 16, 1401-1420. https://doi.org/10.1007/s11368-016-1403-8), its contribution to nitrification is comparatively minor (Kits et al., Kinetic Analysis of a Complete Nitrifier Reveals an Oligotrophic Lifestyle. Nature 2017, 549, 269-272. https://doi.org/10.1038/nature23679).

On the contrary, the enzyme hydroxylamine reductase (HAO), required to complete the nitrite formation process, is an iron-dependent oxidoreductase, so it does not require Cu²⁺ or Zn²⁺ (Hendrich et al., The Active Site of Hydroxylamine Oxidoreductase from Nitrosomonas: Evidence for a New Metal Cluster in Enzymes. J. Am. Chem. Soc. 1994, 116, 11961-11968. https://doi.org/10.1021/ja00105a041).

When the above processes occur in a rapid manner, emission losses of N₂O formed from the oxidation of hydroxylamine are favoured. Furthermore, nitrate (NO₃⁻) would be produced, by oxidation of NO₂⁻, in amounts which, if they exceed the needs of the crops at a given time, are susceptible to leaching, with the consequent contamination of aquifers. Therefore, it is necessary to develop strategies that delay the nitrification process in order to avoid economic losses and loss of efficiency (loss of nitrogen supplied to crops) and to prevent environmental pollution (leaching of NO₃⁻ into aquifers and emission of N₂O, a greenhouse gas, to the atmosphere).

One of the strategies to slow down the rate of the nitrification process is by adding chemical compounds capable of inhibiting the nitrification process to fertilisers, generating so-called stabilised fertilisers. Nitrification inhibitors deactivate the enzyme responsible for the first step of nitrification (where the AMO enzyme is involved), keeping NH₄⁺ in the soil for longer (Gilsanz et al., Development of Emission Factors and Efficiency of Two Nitrification Inhibitors, DCD and DMPP. Agriculture, Ecosystems & Environment 2016, 216, 1-8. https://doi.org/10.1016/j.agee.2015.09.030); Ruser et al., The Effect of Nitrification Inhibitors on the Nitrous Oxide Release from Agricultural Soils-a Review. J. Plant Nutr. Soil Sci. 2015, 178, 171-188. https://doi.org/10.1002/jpln.201400251).

Although the number of compounds described as having nitrification inhibitory properties is large, only a few have been developed as products for commercial use. Among them, dicyandiamide (DCD), nitrapyrin and 3,4-dimethylpyrazole phosphate (DMPP) are included. These compounds have proven effectiveness in reducing nitrogen oxide emissions under different climatic conditions, DMPP being particularly effective. 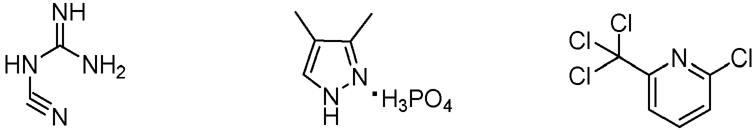 Dicyandiamide 3,4-dimethylpyrazole phosphate Nitrapyrin

Despite the lack of information and some observations not being inconclusive, the nitrification inhibition capacity of these molecules is attributed to their ability to chelate Cu²⁺ cations, which are required by the AMO enzyme to carry out the oxidation of NH₄⁺ to NH₂OH (Subbarao et al., Ruser et al. *supra;* Corrochano-Monsalve al., Impact of Dimethylpyrazole-Based Nitrification Inhibitors on Soil-Borne Bacteria. Science of The Total Environment 2021, 792, 148374. https://doi.org/10.1016/j.scitotenv.2021.148374).

It should be remembered that in soil, chelation is not only the result of the affinity of a chelating agent for the metal, as there are a number of competing reactions that may not favour the chelation of the complex of interest. Therefore, for example, a highly stable chelating agent with Fe³⁺, such as EDTA, may be useless to solve iron deficiencies in calcareous soils, as the high relative abundance of Ca²⁺ relative to Fe³⁺ displaces it from its chelate. Furthermore, metal chelation in an enzyme system is also highly dependent on the accessibility of the chelating agent to the metal present in the enzyme. One example is the urease inhibitor NBPT, which binds to the Ni atoms present in the enzyme due to its high affinity and has a similar accessibility to urea. Although the mechanisms of action may be different (local modification of redox conditions), direct effects on micro-organisms, etc.), these assumptions need to be taken into account when identifying possible modes of action, and, in the context of the present invention, not only the chelation of Cu²⁺ but also of Zn²⁺ and Fe³⁺.

Next, the three most important aforementioned chemical nitrification inhibitors will be discussed: dicyandiamide, 3,4-dimethylpyrazole phosphate and nitrapyrin. For example, US11312667B2 describes these and other chemical nitrification inhibitors.

### • Dicyandiamide (PCD)

Dicyandiamide, also known as cyanoguanidine or DCD, is a chemical compound with the ability to inhibit the first step of nitrification, the oxidation of NH₃/NH₄⁺ to NH₂OH. It is applied by mixing with the nitrogen fertiliser. It has been reported that at concentrations of 200-300 ppm, the production of NO₂⁻ by *Nitrosomonas europea* bacteria can be significantly reduced under *in vitro* conditions. In soil studies, a dose of 8 mg of DCD per kg of soil has been shown to be effective in inhibiting *Nitrosospira* activity in grasslands with animal urine as an NH₄⁺ substrate (Di et al., *supra).* Although some bibliographical references attribute its mechanism of action to its ability to chelate the Cu²⁺ required by the AMO enzyme to oxidise NH₃/NH₄⁺, the mechanism of inhibition at the molecular level has not yet been confirmed. Thanks to the studies carried out, it has been shown that DCD does not kill the bacteria because, although it temporarily inhibits the growth and activity of ammonia/ammonium oxidising bacteria, it has been shown that once DCD is degraded in the soil, these bacteria recover their oxidation activity (Di et al., *supra).* It should be noted that the inhibitory capacity of DCD has been shown to be specific to this transformation, and therefore its use in soil does not affect other microbial communities and other enzymatic activities (Amberger, A., Research on Dicyandiamide as a Nitrification Inhibitor and Future Outlook. Communications in Soil Science and Plant Analysis 1989, 20, 1933-1955. https://doi.org/10.1080/00103628909368195).

However, the use of DCD may cause problems related to their uptake by plants while they remain in the soil before degradation. Therefore, Marsden et al. (Plant Acquisition and Metabolism of the Synthetic Nitrification Inhibitor Dicyandiamide and Naturally-Occurring Guanidine from Agricultural Soils. Plant So/7 2015, 395, 201-214. https://doi.org/10.1007/s11104-015-2549-7) have shown that DCD can be passed to and partially metabolised in grass plants, being a possible vehicle for transmission into the food chain.

### • 3,4-dimethylpyrazole phosphate (DMPP)

3,4-dimethylpyrazole phosphate (DMPP) is a nitrification inhibitor developed by BASF and marketed as ENTEC^{®} since 1999. Like DCD, it is a highly effective inhibitor of the AMO enzyme in the oxidation process of NH₃ or NH₄⁺ to hydroxylamine. It has a higher inhibitory capacity than DCD, therefore the application rate to observe satisfactory inhibition effects is lower (Pasda et al., Effect of Fertilizers with the New Nitrification Inhibitor DMPP (3,4-Dimethylpyrazole Phosphate) on Yield and Quality of Agricultural and Horticultural Crops. Biology and Fertility of Soils 2001, 34, 85-97. https://doi.org/10.1007/s003740100381; Zerulla et al., 3,4-Dimethylpyrazole Phosphate (DMPP) - a New Nitrification Inhibitor for Agricultura and Horticultura. Biology and Fertility of Soils 2001, 34, 79-84. https://doi.org/10.1007/s003740100380). DMPP is decomposed in the soil by micro-organisms through the cleavage of the pyrazole ring releasing CO2. Moreover, it has been shown to remain in the soil for longer than its inhibitory effect (Guardia et al. 15N-Labelled Ammonium Nitrate with or without the New Nitrification Inhibitor DMPSA in an Irrigated Maize Crop. Soil Biology and Biochemistry 2018, 116, 193-202. https://doi.org/10.1016/j.soilbio.2017.10.013), thus being disadvantageous due to its possible uptake by the plant. On the other hand, DMPP has a lower mobility in soil than DCD. Because of this, when applied with an ammonium N-based fertiliser, it remains together with the released NH₄⁺ to provide the inhibitory effect.

### • Nitrapyrin

Nitrapyrin is an organochlorine compound developed by Dow Chemical and sold as N-Serve^{®} in US). It is soluble in most organic solvents but insoluble in water, and also has a high vapour pressure. Despite these disadvantages compared to DMPP and DCD, is a nitrification inhibitor widely used in the US due to its high volatility. Its main method of use has been by injection into the soil (5-10 cm depth) together with anhydrous NH3, particularly in maize fields (Slangen et al., Nitrification Inhibitors in Agriculture and Horticulture: A Literature Review. Fertilizer Research 1984, 5, 1-76. https://doi.org/10.1007/BF01049492). However, due to its high volatility, is not an effective inhibitor as a coating for solid nitrogen fertilisers that are broadcast sown.

Because of the problems that these chemical nitrification inhibitors can cause to the environment due to their persistence therein, studies have emerged proposing the use of biological nitrification inhibitors. These inhibitors consist of chemical compounds exuded by plants or other organisms with the capacity to inhibit nitrification. However, its effectiveness at the agronomic level is not yet well quantified, a problem being the specificity of the plant species that can generate them.

There is still not much information and knowledge about this type of inhibitors, but compounds are known to exist in *Brachiaria* exudates, such as brachylactone, with AMO and HAO inhibitory capacity (Subbarao et al., Evidence for Biological Nitrification Inhibition in Brachiaria Pastures. Proc. Nati. Acad. Sci. U.S.A. 2009, 106, 17302-17307, https://doi.org/10.1073/pnas.0903694106e) and compounds such as methyl 3-(4-hydroxyphenyl)propionate (MHPP) and 5,4'-dihydroxy-7-methoxyflavanone (sakuranetin) are released in sorghum exudates which also affect the AMO and HAO enzymes (Subbarao et al., Biological Nitrification Inhibition (BNI) Activity in Sorghum and Its Characterization. Plant Soil 2013, 366, 243-259. https://doi.org/10.1007/s11104-012-1419-9).

Nitrification inhibition using biodegradable plant extracts has been proposed as a promising alternative to synthetic nitrification inhibitors. Therefore, oils from *Mentha spicata L.* and *Artemisia annua L.* have been shown to inhibit nitrification (Kiran et al., Influence of Natural Essential Oils and Their By-Products as Nitrification Retarders in Regulating Nitrogen Utilization for Japanese Mint in Sandy Loam Soils of Subtropical Central India. Agriculture, Ecosystems & Environment 2003, 94, 237-245. https://doi.org/10.1016/S0167-8809(02)00027-0).

Other candidate nitrification inhibitors of biological origin include phenolic compounds. For example, catechin-containing plant extracts have shown nitrification inhibitory capacity (Castaldi et al., Inhibition of Net Nitrification Activity in a Mediterranean Woodland: Possible Role of Chemicals Produced by Arbutus Unedo. Plant Soil 2009, 315, 273-283. https://doi.org/10.1007/s11104-008-9750-x).

In addition, nitrification inhibitory activity has recently been documented in alpechines (by-products rich in phenolic compounds), indicating that these show a greater effect on nitrification than extracts of Mentha piperita and Artemisia annua. Di Martino et al. in "Effects of Olive Mili Wastewater and Two Natural Extracts as Nitrification Inhibitors on Activity of Nitrifying Bacteria, Soil Nitrate Leaching Loss, and Nitrogen Metabolism of Celery (Apium Graveolens L.). J Plant Growth Regul 2021, 40, 1922-1938", claim that the compounds present in the alpechine are able to reduce the activity of nitrifying bacteria, increase the retention time in the soil of nitrogen from a fertiliser based on (NH₄)₂SO₄ and reduce the leaching of NO₃⁻, with an effectiveness similar to that of DCD (synthetic nitrification inhibitor).

It has also been proposed that caffeic acid, a hydroxycinnamic acid intermediate of lignin biosynthesis, inhibits nitrification by deactivating nitric oxide due to its antioxidant properties (Sauder et al., Nitric Oxide Scavengers Differentially Inhibit Ammonia Oxidation in Ammonia-Oxidizing Archaea and Bacteria. FEMS Microbiology Letters 2016, 363, 1-8. https://doi.org/10.1093/femsle/fnw052).

Taking into account the nature of the compounds mentioned up to this point with nitrification inhibition capabilities, both synthetic and bio-based, certain compounds of microbial origin could have an effect on nitrification. For example, certain bacteria and fungi have a high capacity to produce antioxidant compounds and metabolites with the ability for complexing metal elements or surfactant compounds, which may have effects on other micro-organisms and, specifically, on those involved in nitrification (Nardi et al., Biological Nitrification Inhibition in the Rhizosphere: Determining Interactions and Impact on Microbially Mediated Processes and Potential Applications. FEMS Microbiology Reviews 2020, 44, 874-908. https://doi.org/10.1093/femsre/fuaa037; Chandra et al., Antioxidant Compounds from Microbial Sources: A Review. Food Res. Int. 2020, 129, 108849.

https://doi.org/10.1016/j.foodres.2019.108849; Slama et al., Screening for Fusarium Antagonistic Bacteria from Contrasting Niches Designated the Endophyte Bacillus Halotolerans as Plant Warden Against Fusarium. Front. Microbiol. 2019, 9, 3236. https://doi.org/10.3389/fmicb.2018.03236). For example, CN108794185 attributes to rhamnolipids of microbial origin the ability to inhibit nitrification.

Given the drawbacks of known nitrification inhibitors, it would be desirable to provide alternative nitrification inhibitors to the use of chemical inhibitors.

Therefore, it is an object of the invention to provide a nitrification inhibitor which does not entail the drawbacks of known inhibitors, said nitrification inhibitor being found to be pulcherriminic acid, a compound of biological origin.

Figure 1 shows a diagram for obtaining the pulcherriminic acid used in the present invention.

Figure 2 shows the ¹H-NMR spectrum for pulcherriminic acid obtained according to Figure 1.

Figure 3 shows the ¹³C-NMR (APT) spectrum for pulcherriminic acid obtained according to Figure 1.

Figure 4 shows the UPLC-MS chromatogram of pulcherriminic acid obtained according to Figure 1.

Figure 5 shows the mass spectrum of the peak detected at 14.06 min of the pulcherriminic acid obtained according to Figure 1.

The present inventors have found that, surprisingly, pulcherriminic acid has shown a potent nitrification inhibitory effect both in *in vitro* tests with *Nitrosomonas* bacteria and in *in vivo* soil tests.

According to the literature, pulcherriminic acid or 2,5-dihydroxy-3,6-diisobutylpyrazine-1,4-*N*/*N*'dioxide **(1)** is a precursor compound of a reddish pigment called pulcherrimine **(2)** (Kluyver et al., Pulcherrimin, The Pigment of Candida Pulcherrima. Proceedings of the National Academy of Sciences 1953, 39, 583-593. https://doi.org/10.1073/pnas.39.7.583; MacDonald, J. C., The Structure of Pulcherriminic Acid. Can. J. Chem. 1963, 41, 165-172. https://doi.org/10.1139/v63-021).

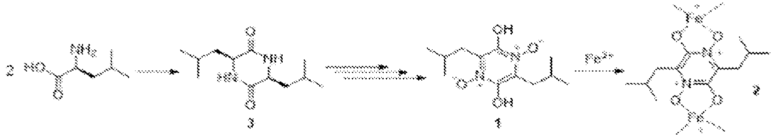

Pulcherriminic acid is biosynthesised by some yeasts of the *Metschnikowia* family and some bacteria of the *Bacillus* family from the cyclic dipeptide derived from L-leucine **(3),** prepared from L-leucine. In the presence of Fe(III), pulcherriminic acid forms a polymeric chelate known as pulcherrimine through a non-enzymatic reaction (Gore-Lloyd et al., Snf2 Controls Pulcherriminic Acid Biosynthesis and Antifungal Activity of the Biocontrol Yeast Metschnikowia Pulcherrima. Mol. Microbiol. 2019, 112, 317-332. https://doi.org/10.1111/mmi.14272; Sipiczki, M., Metschnikowia Pulcherrima and Related Pulcherrimin-Producing Yeasts: Fuzzy Species Boundaries and Complex Antimicrobial Antagonism. Microorganisms 2020, 8, 1029. https://doi.org/10.3390/microorganisms8071029). Therefore, pulcherriminic acid is a compound of biological origin.

As mentioned above, the present invention discloses pulcherriminic acid as a nitrification inhibitor for use thereof in fertilisers containing ammoniacal nitrogen, urea nitrogen or both.

Preferably, pulcherriminic acid is present in fertilisers containing ammoniacal nitrogen, urea nitrogen or both in a weight ratio of 0,5 to 10 % relative to the amount of ammoniacal nitrogen, urea nitrogen, or both ammoniacal nitrogen and urea nitrogen.

In the present invention, fertilisers containing ammoniacal nitrogen, urea nitrogen or both are not particularly limited, and may be selected from ammonium nitrate, ammonium phosphate (monoammonium phosphate, di-ammonium phosphate), urea or ammonium sulphate, or combinations thereof, ammonium sulphate being preferred.

The invention is explained in more detail below according to the following examples.

Synthetic pulcherriminic acid obtained from L-leucine is used in the inhibition assays described here.

### Example 1: Production and characterisation of pulcherriminic acid.

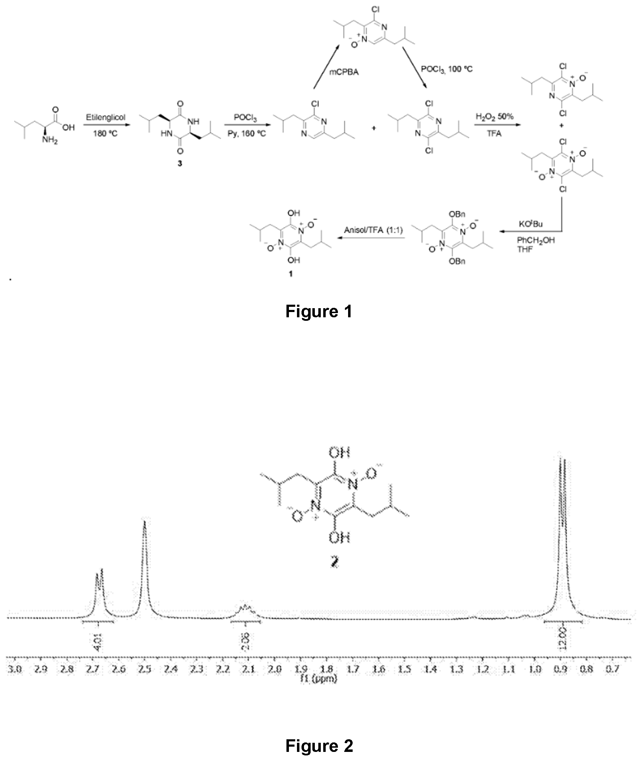

The synthesis diagram of the pulcherriminic acid used is shown in Figure 1.

First, the synthesis of the cyclic dipeptide derived from L-leucine **(3)** was carried out following a thermal procedure described in the literature (Rafiemanzelat et al., Synthesis and Characterization of Hydrolysable Poly(Ether-Urethane-Urea)s Derived from l-Leucine Anhydride Cyclopeptide; a Green Synthetic Method for Monomer. Polymer Degradation and Stability 2010, 95, 901-911. https://doi.org/10.1016/j.polymdegradstab.2010.03.030), yielding a racemic mixture of the cyclic dipeptide derived from L-leucine. This was not a problem as the chirality of the α-carbon of L-leucine is lost in the final product (pulcherriminic acid, **1).** The second step consists of the aromatisation of the 2,5-dichetopiperazine ring to 2,5-dichloropyrazine using POCI3. Using the procedure and conditions described in the literature (Wang et al. Large-Scale Solvent-Free Chlorination of Hydroxy-Pyrimidines, -Pyridines, -Pyrazines and -Amides Using Equimolar POCI3. Molecules 2012, 17, 4533-4544. https://doi.org/10.3390/molecules17044533), a mixture of two compounds was obtained: the desired product, 2,5-dichloro-3,6-diisobutylpyrazine, and an unwanted by-product as a major product, 3-chloro-2,5-diisobutylpyrazine. The by-product could be recovered for transformation thereof into 2,5-dichloro-3,6-diisobutylpyrazine through a two-step process: first step, oxidation of the N in position 1, and second step, chlorination by means of POCI3 >Usui et al., Convergent Synthesis and Structural Confirmation of Phellodonin and Sarcodonin ε. Org. Lett. 2013, 15, 2080-2083. https://doi.org/10.1021/ol400709f).

The next step consists of the oxidation of both nitrogens of the pyrazine ring to give 2,5-dichloro-3,6-diisobutyl-1,4-dioxypyrazine. Through the conditions described in the literature, it could be obtained as a major product by oxidation with hydrogen peroxide in an acid medium (Usui, I. et al., *supra*). Lastly, pulcherriminic acid (1) was obtained with a ≥ 95 % purity by means of a two-step process: aromatic nucleophilic substitution of chlorines with potassium benzyloxide (generated *in situ* by the reaction of benzyl alcohol and *tert*-potassium butoxide) followed by deprotection of the benzyloxy groups in strongly acidic medium (Göktürk et al., Pyrazine Chemistry. Part 13. Preparation and Reactions of Pyrazine N-Oxides Related to Mycelianamide. J. Chem. Soc. Perkin Trans. 1982, 1, 953-959. https://doi.org/10.1039/P19820000953).

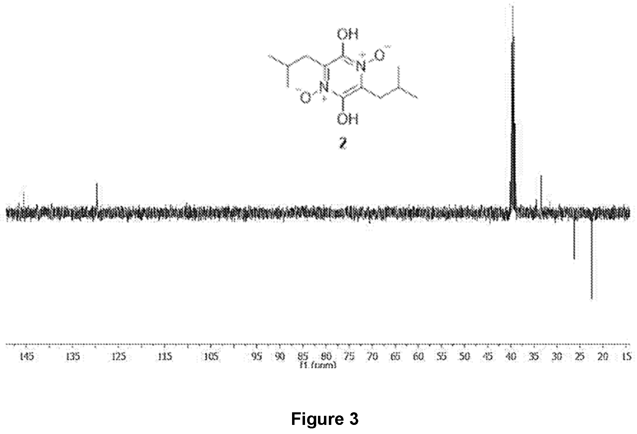

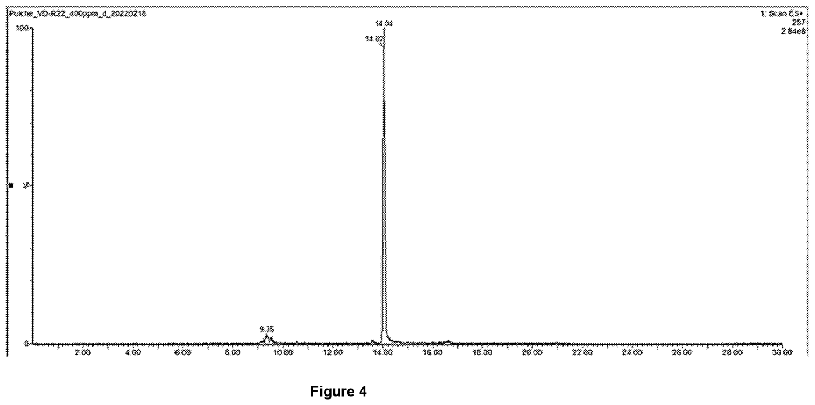

The synthesised pulcherriminic acid (1) was characterised by nuclear magnetic resonance (NMR, Figures 1 and 2) and by ultra-high performance liquid chromatography coupled to mass spectrometry (UPLC-MS), Figures 3 and 4). Its purity was determined by means of CHNS elemental analysis. It was also analysed by means of high resolution mass spectroscopy (HRMS).

The ¹H-NMR and ¹³C-NMR spectra are consistent with those described for pulcherriminic acid (1). The characterisation is shown below:
**¹H-RMN** (DMSO-*d₆*, δ ppm, 400 MHz): 2.67 (d, 4H, J = 7.2 Hz), 2.16-2.06 (m, 2H), 0.89 (d, 12H, *J = Q,Q* Hz).

**¹³C-RMN** (DMSO-*d₆*, δ ppm, 100 MHz): 145.53, 129.63, 33.41, 26.22, 22.39.

By CHNS elemental analysis, a ≥ 95 % purity has been calculated. Next, the results are shown:
- Calculated: C = 54.03 %, H = 7.26 %, N = 10.25 %
- Theoretical: C = 56.24 %, H = 7.87 %, N = 10.93 %

Results obtained by HRMS: found (ESI+) 227.1489 [M + H⁺], C₁₂H₂₁N₂O₄ (Theoretical = 227.1496).

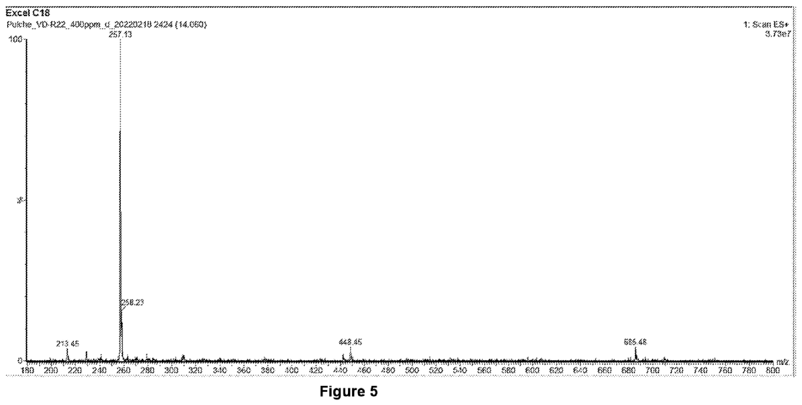

Figures 4 and 5 show the chromatogram obtained by UPLC-MS and the mass spectrum of the detected peak (M + H⁺) with m/z = 257.

### Example 2: Nitrification inhibition capacity of pulcherriminic acid in in vitro tests with Nitrosomonas europaea bacteria

To investigate the nitrification inhibitory capacity of pulcherriminic acid in *in vitro* tests with *Nitrosomonas europaea* bacteria, the pH generated 24 and 48 hours after the application of pulcherriminic acid is measured. The generation of nitric acid by the *Nitrosomonas europaea* bacteria through the nitrification process causes a decrease in pH, so if the pH is maintained it means that nitrification is not taking place or that the nitrification process is inhibited. Table 1 shows the pH of the medium in nitrification tests *in vivo* at different concentrations of pulcherriminic acid. In the control sample (without pulcherriminic acid) it can be seen how the pH drops due to the generation of nitric acid, which indicates that nitrification is taking place.

However, when pulcherriminic acid is added at a concentration of 11.8 ppm, it can be seen that the pH of the medium after 48 hours does not decrease relative to that measured at the initial time, indicating that the nitrification process is inhibited. The same is observed when pulcherriminic acid is added at a dose of 5.9 ppm. Therefore, pulcherriminic acid inhibits the nitrifying activity of *Nitrosomonas europaea* bacteria at a concentration of 5.9 ppm in *in vitro* inhibition assays*.*

**Table 1. Results of the in vitro pulcherriminic acid inhibition assay on Nitrosomonas europaea**

| | Pulcherriminic acid | | |
|---|---|---|---|
| | Control | 11.8 ppm | 5.9 ppm |
| | pH | | |
| Point 0 (t = 0) | 7.94 | 7.88 | 7.91 |
| Point 1 (t = 24 h) | 7.66 | 7.99 | 7.97 |
| Point 2 (t = 48 h) | 6.4 | 8.05 | 7.85 |

In addition, similar to this *in vitro* assay*,* the inhibition capacity of the nitrification process of the pulcherriminic acid precursor, in other words, L-leucine-derived cyclic dipeptide **(3),** was analysed. Table 2 shows that the cyclic dipeptide derived from L-leucine does not inhibit the nitrifying activity of *Nitrosomonas europaea* bacteria even at 2,000 ppm. These results indicate that the structural difference between the two compounds is key to inhibiting the nitrification process.

**Table 2. Results of the in vitro inhibition test of L-leucine-derived cyclic dipeptide in Nitrosomonas europaea**

| | Cyclic dipeptide derived from L-leucine | | | | |
|---|---|---|---|---|---|
| | pH | | | | |
| | Control | 2,000 ppm | 1,000 ppm | 200 ppm | 100 ppm |
| Point 0 (t = 0) | 7.63 | 7.51 | 7.46 | 7.52 | 7.46 |
| Point 1 (t = 24h) | 6.16 | 6.80 | 6.42 | 5.99 | 5.87 |
| Point 2 (t = 48h) | 6.14 | 6.03 | 5.86 | 5.79 | 5.73 |

### Example 3: Nitrification inhibition capacity of pulcherriminic acid in in vivo soil tests.

To analyse the nitrification inhibition activity of pulcherriminic acid in soil, an incubation test was carried out at different doses of nitrogen per hectare and with different inhibitor doses. Three different doses of nitrogen in the form of ammonium sulphate were applied to the same soil type, equivalent to: 100, 200 and 300 kg N/ha. For each dose of nitrogen, different doses of pulcherriminic acid were applied as a nitrification inhibitor, as well as the following controls: nitrogen-free control, nitrogen control and nitrogen controls in the presence of a commercial inhibitor (dicyandiamide). Inhibitor percentages relate to grammes of inhibitor per 100 g of added nitrogen. Table 3 shows the mg of NO₃⁻ per kg of dry soil at the start and 5 days after incubation.

**Table 3. Results of the in vivo inhibition test in soils**

| | | | |
|---|---|---|---|
| | **mg NO₃⁻ / kg dry soil** | **Days** | |
| | **TREATMENT** | 0 | 5 |
| | Control | 1.57 | 6.31 |
| 100 kgN/ha | Control + N | 4.17 | 24.43 |
| | Dicyandiamide 5 % | 3.87 | 19.41 |
| | Pulcherriminic acid 2.5 % | 3.82 | 23.81 |
| | Pulcherriminic acid 5 % | 2.88 | 23.13 |
| | Pulcherriminic acid 10 % | 3.26 | 18.07 |
| 200 kgN/ha | Control + N | 3.45 | 35.76 |
| | Dicyandiamide 5 % | 3.78 | 19.00 |
| | Pulcherriminic acid 2.5 % | 3.38 | 29.83 |
| | Pulcherriminic acid 5 % | 3.30 | 25.46 |
| | Pulcherriminic acid 10 % | 4.24 | 1.22 |
| 300 kgN/ha | Control + N | 3.48 | 31.87 |
| | Dicyandiamide 5 % | 6.99 | 17.73 |
| | Pulcherriminic acid 2.5 % | 3.61 | 31.18 |
| | Pulcherriminic acid 5 % | 3.02 | 17.42 |
| | Pulcherriminic acid 10 % | 3.08 | 5.98 |

As can be seen in the table above, at most nitrogen doses, pulcherriminic acid reduces nitrate formation after 5 days compared to the nitrogen control. At 200 and 300 kg N/ha, the inhibitory capacity of pulcherriminic acid is equal to the inhibitory capacity of dicyandiamide, a commercial nitrification inhibitor of synthetic origin, with the same inhibitor dose. It should be noted that, when 10 % pulcherriminic acid was applied, the nitrification process was totally inhibited after 5 days.

## Claims

1. A nitrification inhibitor for fertilisers containing ammoniacal nitrogen, urea nitrogen or both, wherein the nitrification inhibitor is pulcherriminic acid.

2. The nitrification inhibitor for fertilisers containing ammoniacal nitrogen, urea nitrogen or both according to claim 1 wherein the pulcherriminic acid is present in a weight ratio of 0,5 to 10 % relative to the amount of ammoniacal nitrogen, urea nitrogen, or both ammoniacal nitrogen and urea nitrogen.

3. The nitrification inhibitor for fertilisers containing ammoniacal nitrogen, urea nitrogen or both according to claim 1 wherein the fertilisers containing ammoniacal nitrogen, urea nitrogen or both are selected from ammonium nitrate, ammonium phosphate (monoammonium phosphate, di-ammonium phosphate), urea or ammonium sulphate, or combinations thereof.

4. The nitrification inhibitor for fertilisers containing ammoniacal nitrogen, urea nitrogen or both according to claim 3 wherein said fertiliser is ammonium sulphate.
